# EUROPEAN PATENT APPLICATION

(11) **EP 1 921 058 A1**
(43) Date of publication of application: **14.05.2008**
(21) Application number: 06023432.5
(22) Date of filing: 10.11.2006
(51) Int. Cl.: C07C 45/54, C07C 49/203, C07C 403/16

(54) **Process for the preparation of ionones and vitamin A, vitamin A derivatives, carotenes and carotenoids**

(71) Applicant: DSM IP Assets B.V., 6411 TE Heerlen (NL)
(72) Inventor: The designation of the inventor has not yet been filed
(74) Representative: Pressner, Dietmar

(57) **Abstract**

The present invention relates to a process for the preparation of β-ionones and/or α-ionones of the formulae II and III 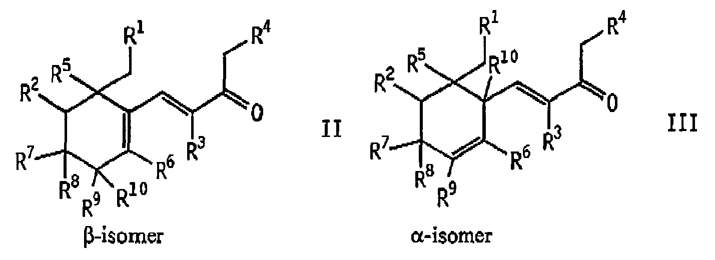 by acid catalyzed ring-closing of ψ-ionones of the formula I 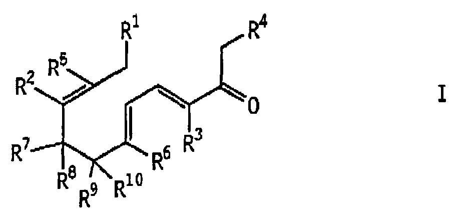 wherein R¹ to R⁷ are independently selected from hydrogen and C₁-C₄-alkyl, R⁸ is selected from hydrogen and C₁-C₄-alkyl and the hydroxyl group, R⁹ and R¹⁰ are independently selected from hydrogen and C₁-C₄-alkyl or R⁹ and R¹⁰ form together the oxygen of a carbonyl group, whereby if R⁹ and R¹⁰ form together the oxygen of a carbonyl group no α-isomer is formed comprising:
a) reacting a ψ-ionone according to formula I in presence of a concentrated Brønsted-acid having a pKs < 3, and an organic solvent that is substantially immiscible with the concentrated acid and with an diluted aqueous solution of said acid;
b) hydrolysis of the reaction product obtained in step a) by addition of water; and phase separation into an organic phase comprising the β-ionone and/or the α-ionone of the formulae II and III and into an aqueous diluted acid phase;

wherein water is added in the hydrolysis step b) in an amount to achieve an acid concentration in said aqueous diluted acid phase of 45 to 65 wt-%, that also can be easily integrated in a process for the preparation of vitamin A, a vitamin A derivative, a carotene or a carotenoid.

## Description

The present invention relates to a process for the preparation of β-ionones and/or α-ionones and to a process for the preparation of vitamin A, vitamin A derivatives, carotenes and carotenoids,

The objective of the industrial synthesis of vitamin A, vitamin A derivatives, carotenes and carotenoids is to build up the structure from readily available components, e.g. from petrochemicals. In β-ionone, a cyclic terpene ketone, there is already available a molecule which contains 13 carbon atoms including the terminal C₆ ring in the configuration corresponding to vitamin A. Consequently all large scale processes for the synthesis of vitamin A or vitamin A derivatives proceed via β-ionone as an intermediate.

The Isler synthesis of 1948, the key step of which is based on the linking of a C₁₄ component formed from β-ionone with a C₆ component, can be regarded up to now as one of the most economically successful process for the total synthesis of vitamin A. In its industrial implementation, this synthesis comprises multiple stages, of which the second consists of the cyclization of ψ-ionone to β-ionone in the presence of sulfuric acid. β-ionone is converted via the glycidic ester synthesis into the C₁₄ component, 2-methyl-4-(2,6,6-trimethyl-1-cyclohexen-1-yl)-2-buten-1-al (the "C₁₄ aldehyde"), which in turn is linked, via a Grignard reaction, with the C₆ component, 3-methyl-pent-2-en-4-yn-1-ol, synthesized in 3 reaction steps. Vitamin A acetate is obtained in crude form from the resulting oxenyne after partial Lindlar hydrogenation, acetylation with acetic anhydride, dehydration and rearrangement. The crude vitamin A acetate is purified by crystallization to form, the commercial end product of the large-scale synthesis. Thereafter the vitamin A acetate can be further reacted to obtain the desired vitamin A derivatives.

The central compound of all vitamin A syntheses is β-ionone which can be obtained from ψ-ionone (6,10-dimethylundeca-3,5,9-trien-2-one) by ring-closure reaction under the action of a strong Bronsted-acid, In this ring-closure reaction also the α-ionone isomer is formed which differs from the β-ionone only by the position of the double bond in the ring. Both isomers are valuable products. For example α-ionone can be used in the perfume industry as an odorant precursor, Thus for a commercially successful process it is desired to obtain an optimal combined yield of β-ionone and α-ionone. In case the focus is on the preparation of vitamin A or vitamin A derivatives via the intermediate β-ionone it is preferred to optimize the β-ionone yield in the above discussed ring-closing reaction,

As described in the prior art section of U.S. Patent 5,453,546 in an industrial-scale plant ψ-ionone is continuously reacted completely with highly concentrated, e.g., 98% sulfuric acid, with β-ionone being obtained in a yield of about 90%. The ψ-ionone is taken from intermediate storage tanks via metering pumps and is dissolved in methylene chloride. The thus-diluted educt, ψ-ionone, is mixed intensively in the reaction zone with sulfuric acid in a weight ratio of about 1:2. The acid is substantially insoluble in methylene chloride so that a two-phase liquid/liquid system forms in which the reaction is initiated at the phase boundary. The heat liberated in the strongly exothermic reaction is removed by pre-cooling the ψ-ionone/methylene chloride stream and intensively cooling in the reactor to maintain a temperature of 0° to 5°C. Since β-ionone in contact with sulfuric acid forms high molecular weight polymeric by-products with increasing reaction time, it is necessary to suppress further reaction. In a so-called quenching stage, the sulfuric acid is diluted by the metered addition of water to a sufficient extent, normally to an about 18% aqueous solution, so that with simultaneous separation of the resulting organic and the acidic aqueous phase the reaction is stopped. The liberated enthalpy of dilution must be removed in this case.

Starting now from this industrial-scale process for the preparation of β-ionone U.S. Patent 5,453,546 addresses the problem of use of chlorinated solvents, as well as the re-use of sulfuric acid that is not consumed in the reaction but functions as a catalyst. But due to the quenching step the initially used highly concentrated sulfuric acid is diluted to a considerably low concentration of about 18% so that re-use of the sulfuric acid would require a high degree of concentration, i.e. from 18% up to about 98%, which is very energy-intensive and costly.

To overcome this problem U.S. Patent 5,453,546 proposes to use a two-phase solvent system under high pressure wherein the first phase comprises an aqueous solution of at least 85 wt-% sulfuric acid, and the second phase comprises liquid carbon dioxide having ψ-ionone dissolved therein to overcome the above described problems,

But in the process described in U.S. Patent 5,453,546 still the addition of water or diluted sulfuric acid is necessary for quenching whereby a final sulfuric acid concentration after quenching of 40 to 50% is obtained.

The process disclosed in U.S. Patent 5,453,546 has several disadvantages. First of all, it adds to the complexity of the entire industrial process to use as a solvent carbon dioxide which requires high pressure and low temperature conditions making the process very costly in terms of investment costs and variable costs, especially energy costs.

Furthermore, when looking into the experimental data provided in U.S. Patent 5,453,546 the total yield of the desired β-ionone is considerably lower than the yield described in the prior art section of U.S. Patent 5,453,546 of the already known industrial process. There is only one example wherein the yield is around 90% for β-ionone. Furthermore, it is described in column 8, lines 52-61, of U.S, Patent 5,453,546 that in order to obtain the desired yield the final acid concentration after quenching should be 45% at most, and in the example a final concentration of 40% was adjusted.

Consequently, in addition to the problems associated with high pressure/low temperature processing, due to the use of carbon dioxide as solvent, the obtained yields are undesirably low, and after quenching sulfuric acid is still in a quite diluted concentration of around 40% so that still a high energy consumption is necessary for concentrating the obtained diluted sulfuric acid to a usable concentration.

DE-A 196 19 557, in order to overcome the above discussed problems of process complexity due to the use of carbon dioxide, suggests to conduct the cyclization of the ψ-ionone and the subsequent quenching or hydrolysis in a substantially adiabatic reaction in one or more mixing pumps having a specific well-defined design and geometry. According to the teaching of DE-A 196 19 557 aromatic aliphatic or cycloaliphatic hydrocarbons, as well as aliphatic chlorinated hydrocarbons can be used as solvent. In the quenching step water is added to obtain a final diluted sulfuric acid concentration of 28.5 to 40%. In the examples the concentration range for the finally obtained diluted sulfuric acid is between 30% and 37%.

But, when looking into the experimental data, the obtained β-ionone yield is still undesirably low with the highest yield of around 85%.

In summary, the teaching of both prior art references, U.S. Patent 5,453,546 and DE-A 196 19 557, despite adding enormous complexity to the process, result in β-ionone yields that are lower than those described for the standard industrial process, and the concentration of the finally obtained diluted sulfuric acid after quenching is still low at 40 or 45% at a maximum, so that re-using the sulfuric acid obtained from the process still needs considerable concentration.

Thus it is the object of the present invention to provide a process for the preparation of α-ionones and/or β-ionones using existing standard production plants to improve the overall efficiency of the process without compromising the overall yield of the final α-ionones and β-ionones product. It is a further object of the present invention to optimize the yield of β-ionones.

It is still a further object of the present invention to integrate the above discussed ring closing step into the overall process for the preparation of vitamin A, vitamin A derivatives, carotenes or carotenoids.

### Summary of the Invention

These objects have been attained by a process for the preparation of β-ionones and/or α-ionones of the formulae II and III 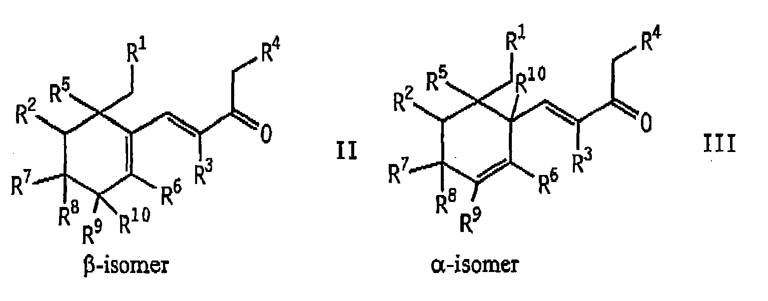 by acid catalyzed ring-closing of ψ-ionones of the formula I 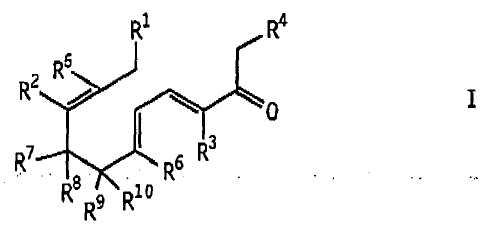 wherein R¹ to R⁷ are independently selected from hydrogen and C₁-C₄-akyl R⁸ is selected from hydrogen and C₁-C₄-alkyl and the hydroxyl group, R⁹ and R¹⁰ are independently selected from hydrogen and C₁-C₄-alkyl or R⁹ and R¹⁰ form together the oxygen of a carbonyl group, whereby if R⁹ and R¹⁰ form together the oxygen of a carbonyl group no α-isomer is formed comprising:
a) reacting a ψ-ionone according to formula I in presence of a concentrated Brønsted-acid having a pKs < 3, and an organic solvent that is substantially immiscible with the concentrated acid and with an diluted aqueous solution of said acid;
b) hydrolysis of the reaction product obtained in step a) by addition of water containing composition; and phase separation into an organic phase comprising the β-ionone and/or the α-ionone of the formulae II and III and into an aqueous diluted acid phase;
wherein water is added in the hydrolysis step b) in an amount to achieve an acid concentration in said aqueous diluted acid phase of 45 to 65 wt-%, and by a process for the preparation of vitamin A, vitamin A derivatives, carotenes or carotenoids by obtaining a β-ionone from the above defined process after isolation and optionally purification of the β-ionone and converting the β-ionone to vitamin A, vitamin A derivatives, carotenes or carotenoids.

The present inventors have surprisingly discovered that despite the above discussed teaching of the prior art water can be added in the quenching or hydrolysis step b) in a amount to obtain an acid concentration in the aqueous diluted acid phase of 45 to 65 wt-%, using standard reaction equipment without compromising the combined yield of α-ionone and β-ionone.

Thereby the overall efficiency of the process can be considerably improved since, compared to the prior art, due to the relatively high acid concentration in the aqueous diluted acid phase less energy is necessary in order to obtain a concentrated acid that can be re-used in the present process or in other processes.

It is even more surprising that this goal could be achieved by using standard solvents known in the art for the ring-closing step starting form pseudo-ionones.

### Detailed Description of the Present Invention

Although the present invention is described in more detail on basis of the reaction of ψ-ionone to β-ionone a person skilled in the art will understand that the same principle will also apply to close homologues of ψ-ionone represented by the above described formula I, but of course, the process of the present invention is particularly advantageous for the preparation of β-ionone which is an important intermediate for the preparation of vitamin A and vitamin A derivatives on an industrial scale. But the process of the present invention can also be applied for the preparation of the corresponding α-isomers for example α-ionone or for making substituted β-ionones as defined by formula II as intermediates for vitamin A derivatives, carotenes or carotenoids. According to a preferred embodiment of the present invention, substituents R¹ to R⁴ and R⁷ to R¹⁰ in formula I and formula II are all hydrogen and R⁵ and R⁶ are methyl, thus representing ψ-ionone and β-ionone, respectively.

The selection of a specific acid is not essential to the present invention. The requirement that has to be fulfilled is that the acid is a strong acid capable of transferring a proton which is defined according to the present invention as a Bronsted-acid having a pKs < 3. Preferably the pks is <0. Furthermore, it is preferred that the acid should be under the reaction conditions non-oxidizing to avoid decomposition of the starting material or valuable products.

The term "concentrated acid" according to present invention is to be understood as to refer to an aqueous solution of the acid in a concentration of at least 90 wt-%. Furthermore, to suppress unwanted side-reactions, the concentration of the acid is preferably at least 95 wt-%, more preferred 95 to 98 wt-%.

Acids that are suitable according to the present invention can be selected from sulfuric acid, phosphoric acids or methane sulfonic acid. According to a preferred embodiment of the present invention the concentrated acid is sulfuric acid having a concentration of at least 95 wt-%, preferably 95 to 98 wt-%. It is most preferred if the acid is concentrated sulfuric acid in a concentration of 98 wt-%.

Furthermore, the selection of a specific solvent is also not critical to the present invention as long as, depending on the selection of the acid, a two-phase reaction system is maintained so that neither the concentrated acid, nor the diluted aqueous solution of said acid that is obtained in the hydrolysis step b), is substantially miscible with the organic solvent employed in the process of the present invention.

By the term "substantially immiscible" according to the present invention, it is meant that during the reaction step a) as well as during the hydrolysis step b) two distinct liquid phases are maintained,

Preferably the organic solvent is selected from hydrocarbons and chlorinated hydrocarbons. But sulfolan is also a suitable solvent.

It is especially preferred if the solvent is selected from chlorinated hydrocarbons, preferably from chloroform and methylene chloride, whereby methylene chloride is most preferred.

But it is also possible to use non-chlorinated solvents, like aliphatic or aromatic hydrocarbons, whereby aliphatic hydrocarbons are more preferred since they lead to fewer unwanted side-products, compared to aromatic hydrocarbons.

Among the aliphatic hydrocarbons hexane and heptane are particularly useful for the present invention.

Furthermore, it is advantageous for the subsequent phase separation that only small amounts of water are added in the hydrolysis to achieve a phase separation with the aqueous phase having the higher density.

This requirement is, e.g., achieved if methylene chloride or hexane is used as solvent, and sulfuric acid is used as acid.

For example, for the most preferred system, i.e. methylene chloride, as organic solvent and sulfuric acid as acid, the requirement of higher density of the diluted aqueous phase is achieved if the sulfuric acid concentration is at least 45 wt-%, as required by the present invention.

The present inventors have further discovered that the overall efficiency of the process can be further improved when using as starting material for the β-ionone synthesis technical ψ-ionone comprising at least 96% in total trans-3, trans-5 and trans-3, cis-5 isomers. By using such a purified starting material the total consumption of fresh concentrated acid, as well as the reaction residue that has to be deposited, can be considerably reduced.

The molar ratio of a ψ-ionone according to formula I, to the strong acid is preferably at least 1:2, more preferred the molar ratio is in the range of about 1:2 to about 1:6, whereby a range of 1:2.1 to 1:4.8 is most preferred.

The ring-closing step a) according to the present invention is preferably conducted at a temperature of -30° C - 30° C, preferred -25° C - 20 °C, especially preferred -20° C -15° C.

In the hydrolysis or quenching step b) any suitable aqueous composition can be used. It is preferred to use water, especially softened water. But it may also be advantageous to employ a diluted aqueous solution of the strong acid used in step a), for example diluted sulfuric acid. Depending on the kind of aqueous composition used in step b) the amount of aqueous composition is calculated in order to adjust the final concentration of acid of the diluted acid obtained in step b to be in the range of 45 to 65 wt-% as defined for the present invention. The hydrolysis step b) is conducted at a temperature of -25°C to 20° C, preferably -15° C to 15° C, mostly preferred -10° C to 10 °C.

The process of the present invention can be conducted as a batch process or as a continuous process, whereby a continuous process, especially for use at an industrial scale, is preferred in order to optimize overall process efficiency.

According to one embodiment in order to conduct step a) of the present invention the ψ-ionone according to formula I is continuously mixed with the organic solvent and is cooled to a temperature of approximately 0°C and is fed continuously into a loop reactor. Simultaneously a molar excess in the amounts described above of a strong Brønsted-acid is fed to the loop reactor. Due to the exothermic nature of the reaction the heat of reaction is removed from the reactor by appropriate cooling means.

Thus, it is a preferred embodiment of the present invention that step a) is performed in a reactor with cooling means in order to remove the heat of reaction.

According to a preferred embodiment in case the focus is on an optimized yield of the β-isomer the reaction product obtained from the ring-closing reaction in step a) is transferred into a post-reaction zone in order to convert the α-isomers into the desired β-isomers. Any suitable reactor type can be used for the post-reaction zone, e.g. a cascade of continuously stirred vessels. Thereby it is important that the residence time in the post-reaction zone is optimized in order to obtain a low concentration of the α-isomer. Depending on the conditions and reactor type used for the post-reaction zone the appropriate residence time can be obtained by routine experimentation.

Thereafter, the reaction product is transferred into a reaction vessel for the hydrolysis step b) which can be also a loop reactor with cooling means, in order to remove the heat of reaction. Thus, also for the hydrolysis step b) of the process of the present invention a reactor with cooling means, in order to remove the heat of reaction, is preferred. Water is added to the reaction product in the hydrolysis step in an amount that results in a final diluted aqueous solution of the strong acid having a concentration of 45 to 65 wt-%. Thereby a two-phase system is formed with an organic phase containing the β-ionone product, and an aqueous phase of the diluted strong acid. The reaction product is transferred into a vessel where the two phases can separate, such as a decanter, then the two phases are continuously removed from the separation vessel. The organic phase containing the β-ionone product is then worked up using usual process steps, like washing and neutralizing the organic phase, to remove the remaining traces of acid, followed by phase separation, removal of the solvent whereby the solvent after optional appropriate purification steps, e.g. by rectification, is recycled into the process, and optional further purification of the raw β-ionone, e.g. by rectification.

The thus obtained β-ionone product can be used as starting material for the production of vitamin A and vitamin A derivatives. The above described preferred process for the preparation of β-ionone is likewise applicable to the preparation of other β-ionones represented by the above Formula II that can be used as starting material for other vitamin A derivatives, carotenes and carotenoids like Zeaxanthin, Astaxanthin and Canthaxanthin.

Consequently, the process of the present invention can be easily incorporated into an industrial-scale process for the production of vitamin A, vitamin A derivatives carotenes and cartenoids.

The aqueous diluted acid phase obtained from the phase separation vessel can also be further worked, up. According to a preferred embodiment the aqueous phase is first fed into a tank where the phase can settle. Any organic material remaining in the diluted acid phase after removal from the phase separation vessel thereby coalesce to form an organic phase. The organic phase can be periodically or continuously removed and is combined with the organic phase from the reaction for further work-up.

Finally, the aqueous diluted acid phase can be concentrated by removing water, e.g. by distillation, so that the acid can be re-used in the present process.

In case, according to a preferred embodiment, sulfuric acid is used in the process of the present invention, the aqueous diluted sulfuric acid obtained in the process can be concentrated to 65 wt-% and then can be used in other processes, or the sulfuric acid can be concentrated to at least 95 wt-% to be re-used in the process of the present invention. Since, according to the present invention in the hydrolysis step b), compared to the prior art, less water is added in order to obtain a diluted acid with a concentration in the range of 45 to 65 wt-% without compromising product yield and using standard reactor equipment, less energy is consumed according to the process of the present invention when recovering the concentrated acid from the aqueous diluted acid-phase obtained in the process of the present invention.

The following Examples are provided to illustrate the present invention, but these examples shall not be construed as to limit the present invention defined in the appending claims.

### Example 1

In a 1.3L flask with cooling jacket equipped with a stirrer, thermometer, cooler and 2 dropping funnels, 240 grams of 95 wt-% sulfuric acid, and 400 grams methylene chloride are added. The resultant pale-yellow emulsion is cooled to -10° while stirring. Then 106 grams of ψ-ionone having a purity of 90.6% were added within 10 minutes, so that the temperature is maintained between -5 and 0°C. After completion the dark-red-brownish reaction product was stirred for additional 15 minutes at a temperature between -10 and -20°C.

Thereafter, water is added dropwise within 15 minutes in an amount to result in a final diluted sulfuric acid concentration as given in Table 1, so that the temperature at the end of the addition is 5°C. Alter two-third of the water addition the cooler was switched off and the cooling liquid was removed from the jacket. Then the reaction mixture was stirred for additional 15 minutes at 5°C and the two-phase system was thereafter allowed to settle. The diluted aqueous sulfuric acid phase was removed and extracted twice with 135 grams of methylene chloride.

The combined methylene chloride solutions were washed with 100 grams of water-Thereafter the pH was adjusted to a pH of 8 to 9 by addition of triethyl amine, followed by washing with 200 grams of water.

The methylene chloride phase was concentrated by evaporating methylene chloride using a rotational evaporator. Thereafter the raw β-ionone is distilled for purification.

In Table 1 the yield of β-ionone, α-ionone and the distillation residue is given.

### Examples 2 - 7

Example 1 was repeated whereby water was added to achieve a final concentration of diluted sulfuric acid, as indicated in Table 1. The resultant yield of β-ionone, α-ionone and the distillation residue is also reported in Table 1.

**Table 1**

| Effect of the final concentration of the diluted sulfuric acid after the hydrolysis step on the yield of β-ionone. | | | | |
|---|---|---|---|---|
| Example | Final concentration of diluted sulfuric acid | Yield in % based on ψ-ionone | | |
| | | β-ionone | α-ionone | distillation residue |
| 1* | 19 | 89.7 | 0.8 | 5.8 |
| 2* | 35 | 88.9 | 1.3 | 6.4 |
| 3* | 40 | 88.5 | 2.8 | 7.5 |
| 4 | 50 | 89.7 | 2.3 | 7.3 |
| 5 | 55 | 89.8 | 2.4 | 6.7 |
| 6 | 60 | 88.9 | 3.8 | 7.4 |
| 7 | 65 | 88.2 | 3.5 | 8.9 |

Thus, it has been shown that within the range of 45 to 65% for the final concentration of the aqueous diluted acid phase, contrary to the teaching of the prior art, the yield of desired final product is approximately the same as for the final concentration of 19%, as previously used in the standard β-ionone process,

Thus, a higher final concentration of the diluted acid can be used without compromising yield, thereby reducing the energy needed for subsequent concentration of the diluted acid for further use or re-use in the process of the present invention.

### Example 8

Example 5 was repeated using instead of methylene chloride, hexane. The yield of β-ionone was 88.0%, the yield of α-ionone 0.6%, and the yield of distillation residue was 4.7%. This Example shows that with hexane similar ,results are obtained as with methylene chloride.

### Example 9 (Comparative)

Example 5 was repeated using diisopropyl ether instead of methylene chloride, thereby forming a single-phase reaction mixture. The combined yield of α- and β-ionone was less than 50%. Example 10 shows that using a solvent that is miscible with the strong acid results in a dramatic drop of the yield of the desired product.

## Claims

1. Process for the preparation of β-ionones and/or α-ionones of the formulae II and III 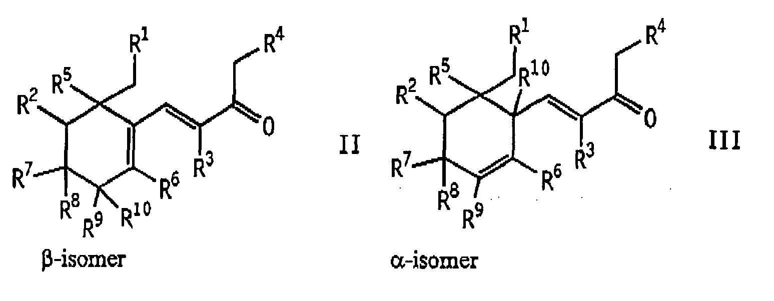 by acid catalyzed ring-closing of ψ-ionones of the formula I 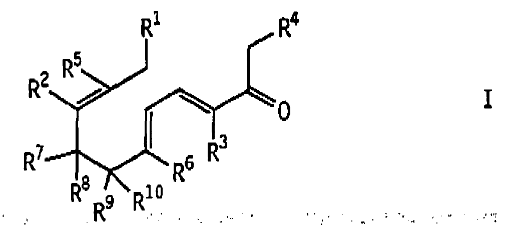 wherein R¹ to R⁷ are independently selected from hydrogen and C₁-C₄-alkyl, R⁸ is selected from hydrogen and C₁-C₄-alkyl and the hydroxyl group, R⁹ and R¹⁰ are independently selected from hydrogen and C₁-C₄-alkyl or R⁹ and R¹⁰ form together the oxygen of a carbonyl group, whereby if R⁹ and R¹⁰ form together the oxygen of a carbonyl group no α-isomer is formed comprising:
a) reacting a ψ-ionone according to formula I in presence of a concentrated Brønsted-acid having a pKs < 3, and an organic solvent that is substantially immiscible with the concentrated acid and with an diluted aqueous solution of said acid;
b) hydrolysis of the reaction product obtained in step a) by addition of a water containing composition; and phase separation into an organic phase comprising the β-ionone and/or the α-ionone of the formulae II and III and into an aqueous diluted acid phase;
wherein water is added in the hydrolysis step b) in an amount to achieve an acid concentration in said aqueous diluted acid phase of 45 to 65 wt-%

2. The process of claim 1, wherein water is added in the hydrolysis step b) in an amount to achieve an acid concentration in the aqueous diluted acid phase of 50 to 60 wt-%, preferably 50 to 55 wt-%,

3. The process of any of the preceding claims, wherein the solvent is selected from hydrocarbons and chlorinated hydrocarbons.

4. The process of claim 3, wherein the solvent is selected from chlorinated hydrocarbons, preferably from chloroform and methylene chloride.

5. The process of claim 3, wherein the solvent is methylene chloride.

6. The process of any of the preceding claims, wherein the density of the aqueous diluted acid phase is higher than the density of the organic phase.

7. The process of any of the preceding claims, wherein the concentrated acid employed in step a) is sulfuric acid having a concentration of at least 95 wt-%, preferably of 95 to 98 wt-%.

8. The process of claim 7, wherein the aqueous diluted sulfuric acid phase is worked-up to obtain a higher concentrated sulfuric acid having a concentration of preferably at least 65 wt-%, or at least 95 wt-%.

9. The process of any of the preceding claims, wherein step a) is conducted at a temperature of-30° C to 30° C.

10. The process of any of the preceding claims, wherein step b) is conducted at a temperature of -25° C to 20° C.

11. The process of any of the preceding claims, wherein R¹ to R⁴ and R⁷ to R¹⁰ all are hydrogen and R⁵ and R⁶ are methyl.

12. The process of claim 11, wherein technical ψ-ionone comprising at least 96 % in total trans-3, trans-5 and trans-3, cis-5 isomers is used as starting compound.

13. The process of any of the preceding claims, further comprising isolation and optionally purification of the β-ionone.

14. The process of any of claims 1-12, further comprising isolation and optionally purification of the α-ionone.

15. A process for the preparation of vitamin A, a vitamin A derivative, a carotene or a carotenoid by
- obtaining a β-ionone by the process of claim 13;
- converting said β-ionone to vitamin A, a vitamin A derivative, a carotene or a carotenoid.
